# FASCICULE DE BREVET EUROPEEN

(11) **EP 2 068 726 B1**
(45) Date de publication et mention de la délivrance du brevet: **06.01.2010**
(21) Numéro de dépôt: 07848300.5
(22) Date de dépôt: 01.10.2007
(51) Int. Cl.: A61B 17/17

(54) **ANCILLAIRE DE VISEE POUR RESURFACAGE DE LA TETE FEMORALE**
HILFSVORRICHTUNG FÜR OBERFLÄCHENERSATZ DES FEMURKOPFES
SIGHTING ANCILLARY DEVICE FOR RESURFACING OF THE FEMORAL HEAD

(30) Priorité: 02.10.2006 FR 0608615; 18.10.2006 US 852417 P
(43) Date de publication de la demande: 17.06.2009
(73) Titulaire: TORNIER, 38330 Saint-Ismier (FR)
(72) Inventeur: SERRAULT, Michel, 61100 Flers (FR); PIRIOU, Philippe, 95270 Belloy En France (FR); COLLARD, Xavier, 7050 Erbisoeul (BE); LAFFARGUE, Philippe, 59110 La Madeleine (FR); FORTHOMME, Jean-Paul, 7030 Saint Symphorien (BE); MIGAUD, Henri, 59000 Lille (FR); PINOIT, Yannick, 59000 Lille (FR); LIZEE, Emmanuel, 38330 Saint Ismier (FR); TORNIER, Alain, 38330 Saint Ismier (FR); RATRON, Yves-Alain, 38000 Grenoble (FR)
(74) Mandataire: Grand, Guillaume
(86) Numéro de dépôt international: PCT/FR2007/001601
(87) Numéro de publication internationale: WO 2008/040874

(56) Documents cités:
- EP-A2- 1 430 844
- WO-A-2004/107993
- WO-A-2005/027755

## Description

La présente invention a trait à un ancillaire de visée pour la pose d'un composant prothétique de resurfaçage de la tête fémorale. Au sens de l'invention, la pose d'un composant prothétique de resurfaçage de la tête fémorale comprend la préparation du fémur et l'implantation effective du composant prothétique sur la tête fémorale.

Une prothèse de resurfaçage de la hanche comprend, de manière classique, un composant de resurfaçage de la tête fémorale. La taille de ce composant, ainsi que son positionnement, sont déterminés au stade préopératoire sur la base d'une analyse d'une ou plusieurs radiographies de la hanche. Un positionnement optimal du composant fémoral correspond à l'alignement de la tige de ce composant sur l'axe central du col fémoral ou en léger valgus, en passant si possible par le centre de la tête fémorale. Un tel positionnement du composant fémoral permet de respecter les critères anatomiques et garantit une bonne tenue mécanique de l'implant. De plus, ce positionnement permet d'éviter des dommages au col fémoral lors de la préparation du fémur, et en particulier lors du fraisage de la tête fémorale, ce qui limite le risque de fracture ultérieure du col fémoral.

Lors de la pose d'un composant fémoral, il est connu d'utiliser un ancillaire pour viser sur la tête fémorale un axe optimal d'implantation du composant fémoral, cet axe ayant été préalablement déterminé sur la base de l'analyse radiographique. La visée de cet axe optimal d'implantation est essentielle car elle détermine la direction de perçage de la tête fémorale et la direction d'approche de l'ensemble des outils de mise en forme du fémur.

WO 2005/027755 décrit un ancillaire de visée selon le préambule de la revendication 1.

US-A-2005/033 290 décrit un ancillaire de visée comprenant un guide de visée dont l'axe de visée est destiné à être aligné sur l'axe optimal d'implantation déterminé lors du planning préopératoire. Cet alignement est obtenu au moyen d'une pince, reliée au guide de visée et prévue pour enserrer le col fémoral. Le positionnement du guide de visée est réglé en ajustant l'inclinaison de la pince par rapport au col fémoral, par alignement d'une tige sur l'axe central du col fémoral vu antérieurement. Un tel ancillaire présente un encombrement important car la tige d'alignement est décalée latéralement par rapport au guide de visée et à la pince. De plus, cet ancillaire de visée impose d'évaluer l'alignement de la tige sur l'axe central du col fémoral, ce qui est délicat et requière des moyens de mesure pour atteindre une bonne précision.

C'est à ces inconvénients qu'entend plus particulièrement remédier l'invention en proposant un ancillaire de visée présentant un encombrement limité et permettant un positionnement aisé et précis de son axe de visée.

A cet effet, l'invention a pour objet un ancillaire de visée pour la pose d'un composant prothétique de resurfaçage de la tête fémorale, comprenant un guide de visée définissant un axe de visée, caractérisé en ce qu'il comprend en outre :
- un support apte à coiffer la tête fémorale et comportant au moins une partie définissant une surface d'enveloppe en portion de sphère sensiblement complémentaire de la tête fémorale, le guide de visée étant fixé sur le support à l'extérieur de la surface d'enveloppe, avec l'axe de visée aligné sur un diamètre de la sphère ou parallèle à un diamètre de la sphère ; et
- au moins un bras de butée sur une partie prédéfinie du col fémoral, chaque bras de butée étant relié au support et ayant une extrémité distale qui est en appui contre la partie prédéfinie correspondante du col fémoral lorsque le support coiffe la tête fémorale avec l'axe de visée aligné sur l'axe central du col fémoral ou en léger valgus par rapport à cet axe.

Selon d'autres caractéristiques avantageuses de cet ancillaire de visée, prises isolément ou selon toutes les combinaisons techniquement possibles :
- l'un au moins des bras de butée est un bras de butée sur la partie intérieure ou sur la partie extérieure du col fémoral ;
- chaque bras de butée comporte un premier segment et un deuxième segment sensiblement perpendiculaires l'un par rapport à l'autre, le premier segment étant fixé sur le support et s'étendant parallèlement à l'axe de visée alors que le deuxième segment comporte l'extrémité distale d'appui sur la partie prédéfinie correspondante du col fémoral ;
- le guide de visée est fixé de manière amovible sur le support ;
- l'ancillaire de visée comprend, pour chaque partie prédéfinie du col fémoral, un jeu de bras de butée de dimensions différentes aptes à être fixés de manière sélective sur le support ;
- l'ancillaire de visée comprend, pour chaque partie prédéfinie du col fémoral, un unique bras de butée apte à être fixé sur le support de manière réglable en coulissement selon une direction sensiblement parallèle et/ou une direction sensiblement perpendiculaire à l'axe de visée ;
- l'ancillaire de visée comprend au moins un autre bras de butée qui est un bras de butée sur la partie antérieure ou sur la partie postérieure du col fémoral ;
- l'ancillaire de visée comprend une broche de fixation du guide de visée sur la tête fémorale, cette broche comportant un corps tubulaire, muni de dents distales et apte à s'emmancher autour du guide de visée ;
- le guide de visée est un tube à section circulaire dont l'axe central forme l'axe de visée, le guide de visée étant percé de lumières de contrôle de l'enfoncement d'un foret introduit à l'intérieur du guide de visée ;
- l'ancillaire de visée comprend un dispositif de vérification de la position de l'axe de visée, ce dispositif comprenant un corps tubulaire apte à s'emmancher autour du guide de visée et une tige propre à palper la partie distale de la tête fémorale sur son pourtour.

Les caractéristiques et avantages de l'invention apparaîtront dans la description qui va suivre d'un mode de réalisation d'un ancillaire de visée selon l'invention, donnée uniquement à titre d'exemple et faite en se référant aux dessins annexés dans lesquels :
- la figure 1 est une vue en perspective éclatée d'un ancillaire de visée conforme à l'invention ;
- la figure 2 est une coupe selon le plan II de la figure 1 ;
- la figure 3 est une vue en perspective selon le même angle de l'ancillaire de visée de la figure 1, lors d'une première étape de visée d'un axe optimal d'implantation pour la pose d'un composant prothétique de resurfaçage de la tête fémorale ;
- la figure 4 est une vue en perspective selon le même angle de l'ancillaire de visée de la figure 1, lors d'une deuxième étape de visée.

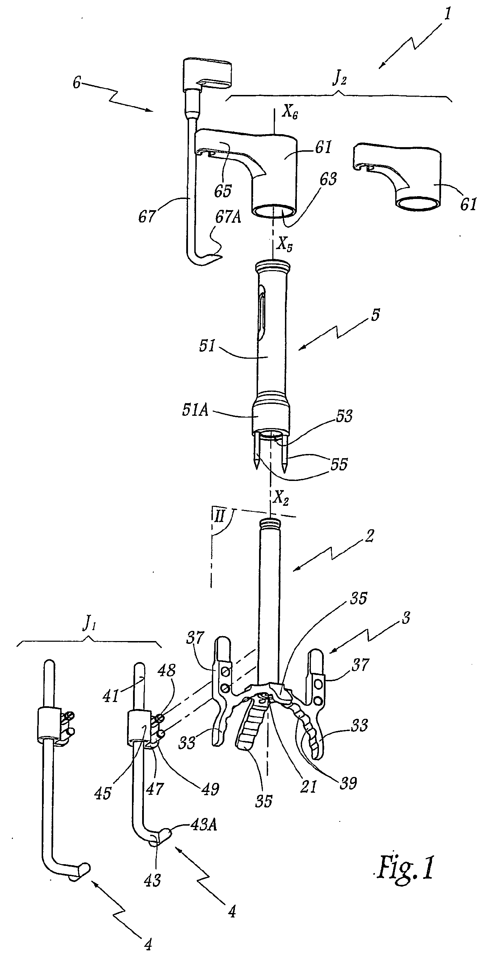

L'ancillaire de visée 1 représenté en éclaté à la figure 1 comprend un guide de visée 2 de forme tubulaire à section circulaire, muni d'un alésage central 21. L'axe central X₂ de l'alésage 21 constitue l'axe de visée de l'ancillaire 1. Le guide de visée 2 est apte à recevoir un foret non représenté dans l'alésage 21, avec l'axe central du foret sensiblement aligné sur l'axe de visée X₂. Le guide de visée 2 constitue alors un guide de perçage pour le foret.

Le guide de visée 2 est destiné à être positionné en regard de la tête fémorale 71 d'un fémur 7 sur lequel doit être implanté un composant de resurfaçage fémoral, avec l'axe de visée X₂ aligné sur un axe optimal d'implantation du composant fémoral. Cet alignement est obtenu grâce à l'ancillaire 1, dont la configuration est adaptée sur la base de paramètres déterminés par exemple lors du planning préopératoire ou statistiquement. Lorsque l'axe de visée X₂ est ainsi positionné, un foret introduit dans l'alésage 21 est à même de percer la tête fémorale 71 selon cet axe optimal d'implantation.

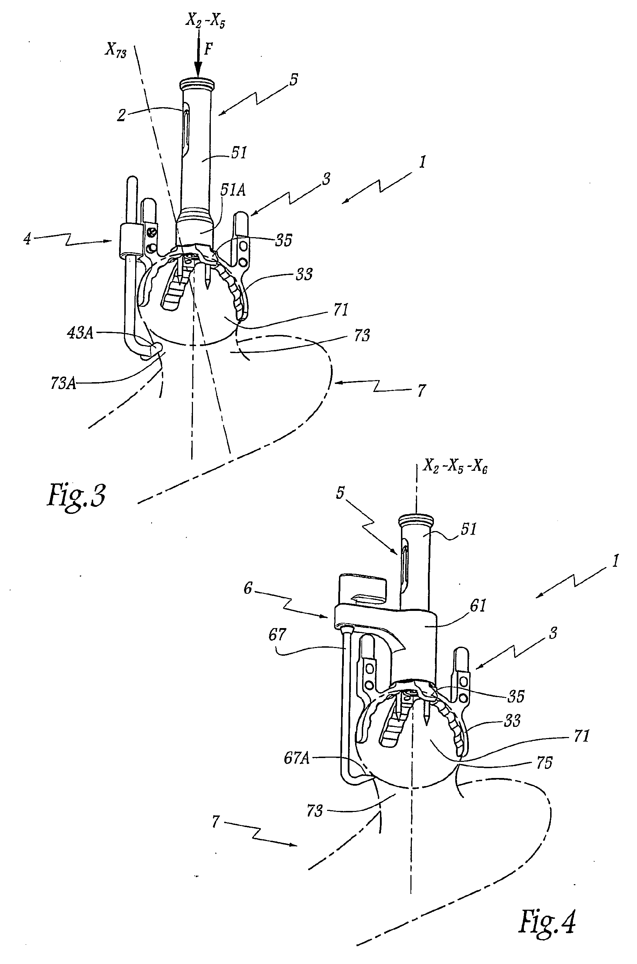

Le guide de visée 2 est fixé rigidement sur un support 3 comportant plusieurs parties 31, 33 et 35, qui définissent ensemble une surface d'enveloppe E₃ de forme hémisphérique sensiblement complémentaire de la tête fémorale 71. En particulier, dans l'exemple représenté, le guide de visée 2 est fixé sur le support 3 avec l'axe de visée X₂ qui passe par le sommet de la demi-sphère formée par la surface d'enveloppe E₃ et forme un diamètre de cette demi-sphère. Le support 3 est apte à coiffer la partie supérieure de la tête fémorale 71, comme visible sur les figures 3 et 4. Lorsque le support 3 coiffe la tête fémorale 71, l'axe de visée X₂ passe sensiblement par le centre de la tête fémorale.

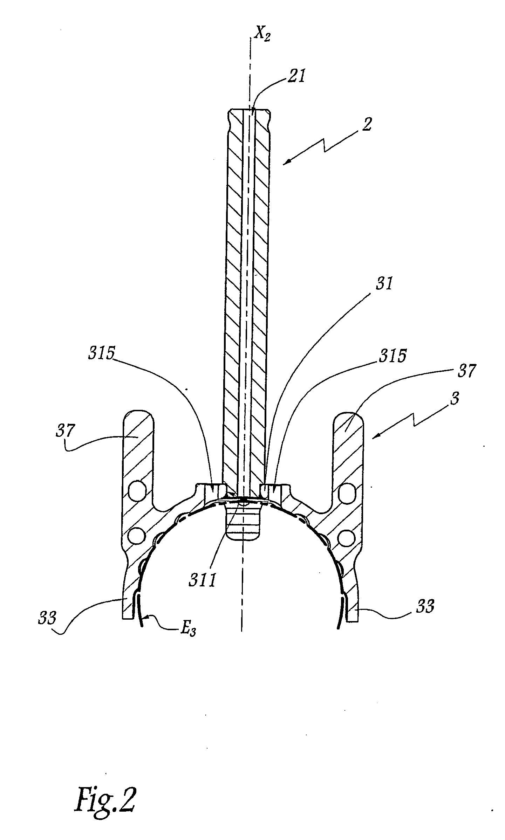

Le support 3 comporte une partie de sommet 31, percée d'un orifice central 311 positionné en correspondance avec l'alésage 21 du guide de visée 2, comme visible sur la figure 2. Quatre pattes 33 et 35 s'étendent à partir de la partie de sommet 31 et sont régulièrement réparties autour de la partie 31. Deux pattes 33, disposées en regard l'une de l'autre, sont destinées à venir en appui respectivement contre les parties intérieure et extérieure de la tête fémorale 71. Les deux autres pattes 35, également disposées en regard l'une de l'autre, sont quant à elles destinées à venir en appui respectivement contre les parties antérieure et postérieure de la tête fémorale 71. Chaque patte 33 ou 35 comporte, sur sa surface concave, des nervures 39 d'accrochage sur la tête fémorale 71.

Dans l'exemple représenté, les deux pattes 33 sont de même longueur, cette longueur étant également celle de la patte 35 destinée à venir en appui contre la partie postérieure de la tête fémorale 71. La patte 35 destinée à venir en appui contre la partie antérieure de la tête fémorale 71 est quant à elle prévue avec une longueur inférieure à celle des autres pattes. Une telle configuration des pattes 33 et 35 permet d'obtenir une bonne stabilité du support 3 lorsqu'il coiffe la tête fémorale 71.

En variante, les deux pattes 35 peuvent être prévues avec une longueur inférieure à celle des pattes 33. Le support 3 est alors moins stable mais plus compact, ce qui réduit l'encombrement de l'ancillaire 1.

L'ancillaire 1 comprend également un bras de butée 4, fixé de manière amovible sur le support 3. Dans l'exemple représenté, une extrémité distale 43A du bras de butée 4 est destinée à venir en appui contre la partie intérieure 73A, ou merckel, du col fémoral 73 du fémur 7. Le support 3 est muni de deux plots 37 de fixation du bras de butée 4, disposés chacun sur la surface convexe d'une patte 33 du support 3 et symétriques l'un de l'autre par rapport à l'axe de visée X₂. Ainsi, le bras de butée 4 est apte à être fixé sur le support 3 au niveau de l'un ou l'autre des plots 37, selon que le fémur 7 est le fémur droit ou gauche du patient. Dans la variante où les deux pattes 35 sont de même longueur, la symétrie du support 3 permet de ne prévoir qu'un seul plot 37 de fixation du bras de butée 4.

Le bras de butée 4 comprend un segment proximal 41 et un segment distal 43, sensiblement perpendiculaires l'un par rapport à l'autre. Le segment proximal 41 est pourvu d'un manchon 45, fixé rigidement au segment 41 et qui se prolonge par une plaque 47 de fixation sur un plot 37. Dans l'exemple représenté, la plaque 47 comporte un clip élastique 48 apte à se verrouiller dans un orifice correspondant prévu dans chaque plot 37. En variante, chaque plot 37 peut comporter un orifice taraudé apte à recevoir une vis passant par un orifice correspondant de la plaque 47. Dans l'exemple représenté, le bras de butée 4 est prévu pour être fixé sur le support 3 avec le segment proximal 41 sensiblement parallèle à l'axe de visée X₂. Pour garantir ce positionnement, la plaque 47 est pourvue d'un pion 49 propre à être inséré dans un orifice correspondant de chaque plot 37. Lorsque le bras de butée 4 est fixé sur le support 3, le segment distal 43, qui comporte l'extrémité distale 43A, s'étend en direction de l'axe de visée X₂, sensiblement perpendiculairement à cet axe.

Les dimensions du bras de butée 4 sont déterminées sur la base de paramètres, obtenus par exemple lors du planning préopératoire ou statistiquement, de sorte que l'axe de visée X₂ est aligné sur un axe optimal d'implantation du composant fémoral lorsque le support 3 coiffe la tête fémorale 71 avec l'extrémité distale 43A du bras de butée 4 en appui contre la partie intérieure 73A du col fémoral 73. Les dimensions du bras de butée 4 dépendent de la morphologie du fémur 7. Dans le mode de réalisation représenté, l'ancillaire 1 est avantageusement prévu avec un jeu J₁ de bras de butée 4 de dimensions différentes, aptes à être fixés de manière sélective sur le support 3. En variante, un ancillaire conforme à l'invention peut comprendre un unique bras de butée 4 apte à être fixé de manière réglable sur le support 3. Notamment, ce réglage peut être réalisé en faisant coulisser le bras de butée 4 selon une première direction sensiblement parallèle à l'axe de visée X₂, par exemple par coulissement du segment 41 par rapport au manchon 45, et selon une deuxième direction sensiblement perpendiculaire à l'axe de visée X₂.

Comme visible sur la figure 1, l'ancillaire 1 comprend également une broche de fixation 5 comportant un corps 51 de forme tubulaire à section circulaire, muni à son extrémité distale 51A de dents d'impaction 55. La broche 5 comporte un alésage central 53, centré sur un axe X₅ et sensiblement complémentaire des dimensions extérieures du guide de visée 2. Ainsi, la broche 5 est apte à s'emmancher autour du guide de visée 2 avec les axes X₂ et X₅ confondus. La partie distale 51A du corps 51 est prévue pour venir en appui contre la partie de sommet 31 du support 3. Des orifices 315 sont en outre prévus dans la partie 31 pour le passage des dents distales 55 de la broche 5.

Enfin, l'ancillaire 1 comprend un satellite 6 de vérification de la position de l'axe de visée X₂ par rapport à la tête fémorale 71. Le satellite 6 comporte un corps 61 de forme tubulaire à section circulaire, muni d'un alésage central 63 d'axe X₆. L'alésage 63 est complémentaire des dimensions extérieures du corps 51 de la broche 5. Ainsi, le corps 61 du satellite 6 est apte à s'emmancher autour du corps 51 de la broche 5, les axes X₅ et X₆ étant alors confondus. Lorsque le guide de visée 2, la broche 5 et le satellite 6 sont tous trois emmanchés les uns par rapport aux autres, comme visible à la figure 4, les trois axes X₂, X₅ et X₆ sont confondus et le corps 61 du satellite 6 vient en appui contre les extrémités proximales des pattes 33 et 35 du support 3.

Le satellite 6 est pourvu d'une tige 67 dont une extrémité distale 67A est propre à palper la partie distale de la tête fémorale à préparer, notamment par fraisage, pour l'implantation du composant de surfaçage fémoral. La tige 67 est reliée au corps 61 au niveau d'une branche latérale 65 du corps 61 tubulaire. Le corps 61 est prévu pour pouvoir être tourné, lorsqu'il est dans la position de la figure 4, autour des axes X₂, X₅ et X₆ confondus. Ainsi, l'extrémité distale 67A de la tige 67 est à même de palper la tête fémorale 71 sur tout son pourtour.

De manière connue en soi, les dimensions du satellite 6 sont ajustées au stade préopératoire pour simuler la taille choisie du composant de resurfaçage fémoral. Dans l'exemple représenté, l'ancillaire 1 est prévu avec un jeu J₂ de corps 61 de satellite de dimensions différentes, aptes à s'emmancher de manière sélective autour de la broche 5 et du guide de visée 2, alors que la tige 67 est unique et apte à coopérer avec chaque corps 61 du jeu J₂ de corps de satellite.

L'utilisation de l'ancillaire de visée 1 est la suivante :
Tout d'abord, on fixe le bras de butée 4 du jeu J₁ ayant des dimensions adaptées, sur le support 3, au niveau du plot de fixation 37 approprié. On emmanche alors la broche 5 autour du guide de visée 2, avec les dents d'impaction 55 décalées par rapport aux orifices de passage 315 de la partie 31 du support 3, de telle sorte qu'elles ne dépassent pas au-delà de la surface concave de la partie 31. Dans cette configuration, l'ancillaire 1 est apte à être déplacé librement sur la tête fémorale 71.

Lorsque l'ancillaire 1 est dans la configuration décrite ci-dessus, on positionne l'ancillaire 1 par rapport au fémur 7. Plus spécifiquement, on coiffe la tête fémorale 71 avec le support 3. L'axe de visée X₂ passe alors sensiblement par le centre de la tête fémorale 71. Ensuite, on incline le support 3 sur la tête fémorale 71 jusqu'à ce que l'extrémité distale 43A du bras de butée 4 vienne en appui contre la partie intérieure 73A du col fémoral 73. Dans cette position, visible à la figure 3, l'axe de visée X₂ est sensiblement aligné sur un axe optimal d'implantation du composant de resurfaçage fémoral, à savoir en léger valgus par rapport à l'axe central X₇₃ du col fémoral 73 et passant par le centre de la tête fémorale 71.

On immobilise alors l'ancillaire 1 sur la tête fémorale 71, de manière à ce que l'axe de visée X₂ soit fixé en alignement sur cet axe optimal d'implantation. Pour cela, on tourne le corps 51 de la broche de fixation 5 par rapport au guide de visée 2 et au support 3, autour des axes X₂ et X₅ confondus, de manière à introduire les dents distales 55 de la broche 5 dans les orifices de passage 315 de la partie 31. On exerce alors un effort F de poussée sur l'extrémité proximale du corps 51 de la broche 5 de manière à impacter les dents 55 dans la partie supérieure de la tête fémorale 71, comme montré sur la figure 3.

On contrôle ensuite la position de l'axe de visée X₂ au moyen du satellite 6 de vérification, de manière à obtenir une bonne adaptation ultérieure du composant de resurfaçage fémoral sur la tête fémorale 71. A cet effet, on désolidarise le bras de butée 4 par rapport au support 3 et on emmanche le corps 61 du satellite 6 autour de la broche de fixation 5. On atteint alors la configuration de l'ancillaire 1 visible à la figure 4. Dans cette configuration, on fait tourner le corps 61 du satellite 6 autour des axes X₂, X₅ et X₆ confondus, de sorte que l'extrémité distale 67A de la tige 67 tourne autour de la jonction 75 entre la tête fémorale 71 et le col fémoral 73. Lorsque l'axe de visée X₂ est correctement positionné, la rotation du corps 61 et de la tige 67 est réalisable sans blocage. Si tel n'est pas le cas, il existe un risque d'endommagement du col fémoral 73 lors des étapes ultérieures de préparation du fémur 7. Un repositionnement de l'axe de visée X₂ doit alors être envisagé.

Lorsque la bonne adaptation du composant de resurfaçage fémoral sur la tête fémorale 71 a été validée au moyen du satellite 6 de vérification, on introduit un foret, non représenté sur les figures, dans l'alésage central 21 du guide de visée 2 et on entraîne le foret en rotation de manière à percer la tête fémorale 71 selon l'axe de visée X₂.

Une fois le perçage réalisé, on retire l'ancillaire de visée de la tête fémorale 71, en désassemblant ses éléments constitutifs. Les étapes ultérieures de mise en forme du fémur 7 et d'implantation du composant de resurfaçage fémoral sont bien connues de l'homme du métier et sont effectuées dans l'alignement du perçage réalisé grâce à l'ancillaire de visée 1 conforme à l'invention.

Comme il ressort des étapes décrites ci-dessus, l'ancillaire de visée 1 selon l'invention permet un positionnement aisé et précis de l'axe de visée X₂ selon un axe optimal d'implantation, à savoir en alignement sur l'axe central X₇₃ du col fémoral 73 ou en léger valgus par rapport à cet axe, en passant par le centre de la tête fémorale 71. En effet, la forme hémisphérique du support 3 permet de trouver aisément le centre de la tête fémorale 71. Le bras de butée 4 permet quant à lui de positionner de manière automatique l'axe de visée X₂ selon un axe optimal d'implantation, c'est-à-dire en alignement ou en léger valgus par rapport à l'axe central X₇₃ du col fémoral 73. De plus, ce positionnement optimal de l'axe de visée X₂ peut être fixé de manière sûre par impaction des dents distales 55 de la broche de fixation 5 dans la tête fémorale 71. Enfin, grâce au satellite 6 de vérification, une bonne adaptation du composant de resurfaçage fémoral sur la tête fémorale 71 est systématiquement vérifiée, préalablement au perçage du fémur 7.

En outre, un tel ancillaire de visée 1 est compact, ce qui est très avantageux dans le cadre d'une intervention chirurgicale de resurfaçage de la hanche. Enfin, un tel ancillaire de visée 1 est adaptable à différentes morphologies de fémurs 7 grâce au jeu J₁ de bras de butée 4 de dimensions différentes ou, en variante, à la fixation réglable d'un bras de butée 4 unique par rapport au support 3.

L'invention n'est pas limitée à l'exemple décrit et représenté. En particulier, le bras de butée 4 peut être destiné à venir en appui contre la partie extérieure du col fémoral 73 au lieu de la partie intérieure 73A du col. En outre, un ancillaire de visée conforme à l'invention peut comprendre deux bras de butée 4, destinés à venir en appui respectivement contre une partie intérieure ou extérieure du col fémoral 73 et contre une partie antérieure ou postérieure du col fémoral 73. La combinaison de tels bras de butée 4 sensiblement perpendiculaires l'un par rapport à l'autre permet le positionnement de l'axe de visée X₂ à la fois dans le plan frontal et dans le plan sagittal.

Selon une autre variante non représentée de l'invention, l'ancillaire de visée 1 peut être modulaire, le guide de visée 2 étant alors fixé de manière amovible sur le support 3 et non monobloc avec le support 3 comme dans l'exemple représenté. Cette variante permet un désassemblage et un retrait facilités de l'ancillaire après le perçage de la tête fémorale 71.

Par ailleurs, le guide de visée 2 peut être pourvu de lumières non représentées de contrôle de l'enfoncement d'un foret de perçage de la tête fémorale 71. En particulier, le foret de perçage peut comprendre un marquage qui, lorsqu'il vient en coïncidence avec les lumières du guide de visée 2, indique un enfoncement optimal du foret.

Enfin, le guide de visée 2 peut être positionné sur le support 3 par l'intermédiaire d'une pièce excentrée, de sorte que l'axe de visée X₂ est parallèle à un diamètre de la demi-sphère formée par le support 3. Une telle solution permet de compenser un décalage éventuel de la tête fémorale 71 par rapport à l'axe central X₇₃ du col fémoral 73.

## Revendications

1. Ancillaire de visée (1) pour la pose d'un composant prothétique de resurfaçage de la tête fémorale, comprenant un guide de visée (2) définissant un axe de visée (X₂), en outre :
- un support (3) apte à coiffer la tête fémorale (71) comprenant le guide de visée (2) étant fixé sur le support (3) ; et
- au moins un bras (4) de butée sur une partie prédéfinie (73A) du col fémoral (73), chaque bras de butée (4) étant relié au support (3) et ayant une extrémité distale (43A) qui est en appui contre la partie prédéfinie correspondante du col fémoral (73) lorsque le support coiffe la tête fémorale avec l'axe de visée (X₂) aligné sur l'axe central (X₇₃) du col fémoral ou en léger valgus par rapport à cet axe, **caractérisé en ce que** le support comporte au moins une partie (31, 33, 35) définissant une surface d'enveloppe (E₃) en portion de sphère sensiblement complémentaire de la tête fémorale (71) et le guide de visée est fixé à l'extérieur de la surface d'enveloppe (E₃), avec l'axe de visée (X₂) aligné sur un diamètre de la sphère ou parallèle à un diamètre de la sphère.

2. Ancillaire de visée selon la revendication 1, **caractérisé en ce que** l'un au moins des bras de butée (4) est un bras de butée sur la partie intérieure (73A) ou sur la partie extérieure du col fémoral.

3. Ancillaire de visée selon l'une quelconque des revendications précédentes, **caractérisé en ce que** chaque bras de butée (4) comporte un premier segment (41) et un deuxième segment (43) sensiblement perpendiculaires l'un par rapport à l'autre, le premier segment étant fixé sur le support (3) et s'étendant parallèlement à l'axe de visée (X₂) alors que le deuxième segment comporte l'extrémité distale (43A) d'appui sur la partie prédéfinie correspondante du col fémoral (73).

4. Ancillaire de visée selon l'une quelconque des revendications précédentes, **caractérisé en ce que** le guide de visée (2) est fixé de manière amovible sur le support (3).

5. Ancillaire de visée selon l'une quelconque des revendications 1 à 4, **caractérisé en ce qu'**il comprend, pour chaque partie prédéfinie du col fémoral (73), un jeu (J₁) de bras de butée (4) de dimensions différentes aptes à être fixés de manière sélective sur le support (3).

6. Ancillaire de visée selon l'une quelconque des revendications 1 à 4, **caractérisé en ce qu'**il comprend, pour chaque partie prédéfinie du col fémoral (73), un unique bras de butée (4) apte à être fixé sur le support (3) de manière réglable en coulissement selon une direction sensiblement parallèle et/ou une direction sensiblement perpendiculaire à l'axe de visée (X₂).

7. Ancillaire de visée selon l'une quelconque des revendications précédentes, **caractérisé en ce qu'**il comprend au moins un autre bras de butée (4) qui est un bras de butée sur la partie antérieure ou sur la partie postérieure du col fémoral (73).

8. Ancillaire de visée selon l'une quelconque des revendications précédentes, **caractérisé en ce qu'**il comprend une broche de fixation (5) du guide de visée (2) sur la tête fémorale (71), cette broche comportant un corps tubulaire (51), muni de dents distales (55) et apte à s'emmancher autour du guide de visée (2).

9. Ancillaire de visée selon l'une quelconque des revendications précédentes, **caractérisé en ce que** le guide de visée (2) est un tube à section circulaire dont l'axe central forme l'axe de visée (X₂), le guide de visée étant percé de lumières de contrôle de l'enfoncement d'un foret introduit à l'intérieur du guide de visée.

10. Ancillaire de visée selon l'une quelconque des revendications précédentes, **caractérisé en ce qu'**il comprend un dispositif (6) de vérification de la position de l'axe de visée (X₂), ce dispositif comprenant un corps tubulaire (61) apte à s'emmancher autour du guide de visée (2) et une tige (67) propre à palper la partie distale de la tête fémorale (71) sur son pourtour.

## Claims

1. A centering jig (1) for positioning a prosthetic component for resurfacing the head of the femur, the jig comprising a centering guide (2) defining a centering axis (X₂), further:
· a support (3) suitable for fitting on the head (71) of the femur, the centering guide (2) being fastened to the support (3); and
· at least one abutment arm (4) for coming into abutment against a predefined portion (73A) of the neck (73) of the femur, each abutment arm (4) being connected to the support (3) and having a distal end (43A) that bears against the corresponding predefined portion of the neck (43) of the femur when the support is fitted on the head of the femur with the centering axis (X₂) aligned on the central axis (X₇₃) of the neck of the femur or with a small amount of valgus relative to said axis,
**characterized in that** the support comprises at least one portion (31, 33, 35) defining an envelope surface (E₃) forming a portion of a sphere substantially complementary to the head (71) of the femur and the centering guide is fastened on the outside of the envelope surface (E₃), with the centering axis (X₂) aligned on a diameter of the sphere or parallel to a diameter of the sphere.

2. A centering jig according to claim 1, **characterized in that** at least one of the abutment arms (4) is an arm for coming into abutment against the internal portion (73A) or the external portion of the neck of the femur.

3. A centering jig according to either preceding claim, **characterized in that** each abutment arm (4) comprises first and second segments (41 and 43) that are substantially perpendicular to each other, the first segment being fastened to the support (3) and extending parallel to the centering axis (X₂), while the second segment has the distal end (43A) for bearing against the corresponding predefined portion of the neck (73) of the femur.

4. A centering jig according to any preceding claim, **characterized in that** the centering guide (2) is releasably fastened to the support (3).

5. A centering jig according to any one of claims 1 to 4, **characterized in that**, for each predefined portion of the neck (73) of the femur, includes a set (J₁) of abutment arms (4) of different dimensions and suitable for being fastened in selective manner to the support (3).

6. A centering jig according to any one of claims 1 to 4, **characterized in that**, for each predefined portion of the neck (73) of the femur, it includes a single abutment arm (4) suitable for being fastened to the support (3) in adjustable manner to slide along a direction that is substantially parallel and/or a direction that is substantially perpendicular to the centering axis (X₂).

7. A centering jig according to any preceding claim, **characterized in that** it includes at least one other abutment arm (4) that is an arm for coming into abutment against the anterior portion or the posterior portion of the neck (73) of the femur.

8. A centering jig according to any preceding claim, **characterized in that** it includes a fastener pin (5) for fastening the centering guide (2) on the head (71) of the femur, said pin comprising a tubular body (51) provided with distal teeth (55) and suitable for being engaged around the centering guide (2).

9. A centering jig according to any preceding claim, **characterized in that** the centering guide (2) is a circular-section tube of central axis forming the centering axis (X₂), the centering guide being pierced by slots for monitoring the extent to which a drill bit inserted inside the centering guide has advanced.

10. A centering jig according to any preceding claim, **characterized in that** it includes a device (6) for verifying the position of the centering axis (X₂), said device comprising a tubular body (61) suitable for engaging around the centering guide (2) and a rod (71) suitable for feeling the distal portion of the head (71) of the femur around its periphery.

## Patentansprüche

1. Hilfsvorrichtung (1) zum Einsetzen einer prothetischen Komponente für den Oberflächenersatz des Femurkopfes, umfassend eine Ausrichtungsführung (2), die eine Ausrichtungsachse (X₂) definiert, und zudem umfassend:
- einen Träger (3), der so gestaltet ist, dass er den Femurkopf (71) bedeckt, wobei die Ausrichtungs-führung (2) auf dem Träger (3) befestigt ist, und
- mindestens einen Arm (4) zum Anschlagen an mindestens einen festgelegten Abschnitt (73A) des Femurhalses (73), wobei jeder Anschlagarm (4) mit dem Träger (3) verbunden ist und ein distales Ende (43A) besitzt, das auf dem festgelegten, zum Femurhals (73) gehörenden Abschnitt aufliegt, wenn der Träger den Femurkopf bedeckt, wobei die Ausrichtungsachse (X₂) mit der Mittelachse (X₇₃) des Femurhalses ausgerichtet ist oder leicht schief zu dieser Achse verläuft, **dadurch gekennzeichnet, dass** der Träger mindestens einen Abschnitt (31, 33, 35) aufweist, der eine teilkugelförmige Umhüllungsoberfläche (E₃) definiert, die im Wesentlichen komplementär zum Femurkopf (71) ist, und die Ausrichtungsführung auf der Außenseite der Umhüllungsoberfläche (E₃) befestigt ist, wobei die Ausrichtungsachse (X₂) auf einem Kugeldurchmesser oder parallel zu einem Kugeldurchmesser ausgerichtet ist.

2. Hilfsvorrichtung nach Anspruch 1, **dadurch gekennzeichnet, dass** mindestens einer der Anschlagarme (4) ein Arm ist, der auf dem Innenabschnitt (73A) oder dem Außenabschnitt des Femurhalses anschlägt.

3. Hilfsvorrichtung nach einem der vorher-gehenden Ansprüche, **dadurch gekennzeichnet, dass** jeder Anschlagarm (4) ein erstes Segment (41) und ein zweites Segment (43) trägt, die im Wesentlichen zueinander senkrecht stehen, wobei das erste Segment auf dem Träger (3) befestigt ist und parallel zur Ausrichtungsachse (X₂) verläuft, während das zweite Segment das distale Ende (43A) trägt, das auf dem festgelegten, zum Femurhals (73) gehörenden Abschnitt aufliegt.

4. Hilfsvorrichtung nach einem der vorher-gehenden Ansprüche, **dadurch gekennzeichnet, dass** die Ausrichtungsführung (2) am Träger (3) abnehmbar angebracht ist.

5. Hilfsvorrichtung nach einem der Ansprüche 1 bis 4, **dadurch gekennzeichnet, dass** sie für jeden festgelegten Abschnitt des Femurhalses (73) einen Satz (J₁) von Anschlagarmen (4) mit unterschiedlichen Abmessungen aufweist, die dafür ausgelegt sind, selektiv am Träger (3) befestigt zu werden.

6. Hilfsvorrichtung nach einem der Ansprüche 1 bis 4, **dadurch gekennzeichnet, dass** sie für jeden festgelegten Abschnitt des Femurhalses (73) einen einzigen Anschlagarm (4) umfasst, der dafür ausgelegt ist, am Träger (3) derart befestigt zu werden, dass er in einer Richtung im Wesentlichen parallel und/oder in einer Richtung im Wesentlichen senkrecht zur Ausrichtungsachse (X₂) gleitend eingestellt werden kann.

7. Hilfsvorrichtung nach einem der vorher-gehenden Ansprüche, **dadurch gekennzeichnet, dass** sie mindestens einen anderen Anschlagarm (4) umfasst, bei dem es sich um einen Arm handelt, der auf dem anterioren oder dem posterioren Abschnitt des Femurhalses (73) anschlägt.

8. Hilfsvorrichtung nach einem der vorher-gehenden Ansprüche, **dadurch gekennzeichnet, dass** sie einen Stift (5) zur Befestigung der Ausrichtungsführung (2) am Femurkopf (71) umfasst, wobei dieser Stift einen röhrenförmigen Körper (51) hat, der mit distalen Zähnen (55) ausgestattet ist, und dafür ausgelegt ist, um die Ausrichtungsführung (2) herum eingesteckt zu werden.

9. Hilfsvorrichtung nach einem der vorher-gehenden Ansprüche, **dadurch gekennzeichnet, dass** die Ausrichtungsführung (2) eine Röhre mit kreisförmigem Querschnitt ist, deren Mittelachse die Ausrichtungs-achse (X₂) bildet, wobei in die Ausrichtungsführung Löcher gebohrt sind zur Kontrolle des Absenkens eines Bohrers, der in das Innere der Ausrichtungsführung eingebracht wird.

10. Hilfsvorrichtung nach einem der vorher-gehenden Ansprüche, **dadurch gekennzeichnet, dass** sie eine Vorrichtung (6) zur Überprüfung der Position der Ausrichtungsachse (X₂) umfasst, wobei diese Vorrichtung einen röhrenförmigen Körper (61), der dafür ausgelegt ist, um die Ausrichtungsführung (2) herum gelegt zu werden, und einen Schaft (67) umfasst, der dafür geeignet ist, den distalen Abschnitt des Femurkopfes (71) an seinem Umfang abzutasten.
